# EUROPÄISCHE PATENTSCHRIFT

(11) **EP 2 687 236 B1**
(45) Veröffentlichungstag und Bekanntmachung des Hinweises auf die Patenterteilung: **18.03.2015**
(21) Anmeldenummer: 13177246.9
(22) Anmeldetag: 19.07.2013
(51) Int. Cl.: A61L 2/08, H01J 33/04, B65B 55/02

(54) **Verfahren und Vorrichtung zum Sterilisieren von Behältnissen mit Kühlluftentnahme aus dem Sterilraum**
Method and device for sterilising containers with cooling air extraction from the sterile area
Procédé et dispositif de stérilisation de récipients avec prélèvement d'air de refroidissement hors de la chambre stérile

(30) Priorität: 19.07.2012 DE 102012106555
(43) Veröffentlichungstag der Anmeldung: 22.01.2014
(73) Patentinhaber: Krones AG, 93073 Neutraubling (DE)
(72) Erfinder: Müller, Holger, 93073 Neutraubling (DE)
(74) Vertreter: Bittner, Bernhard

(56) Entgegenhaltungen:
- EP-A1- 1 160 799
- EP-A1- 1 982 920
- EP-A1- 2 161 202
- WO-A1-2009/144114
- US-A1- 2007 145 304

## Beschreibung

Die vorliegende Erfindung bezieht sich auf ein Verfahren und eine Vorrichtung zum Sterilisieren von Behältnissen.

Die Druckschriften WO 2009/144114 A1, EP 1 982 920 A1 und US 2007/145304 A1 offenbaren jeweils Desinfektionsapparate und -verfahren für Behälter unter Verwendung von Elektronenstrahlen, wobei das Elektronenaustrittsfenster durch ein Fluid gekühlt wird.

Aus dem Stand der Technik ist weiter bekannt, dass bei der Herstellung von Flaschen - insbesondere von Kunststoffflaschen - oftmals Sterilisationsvorgänge erforderlich sind, insbesondere wenn bestimmte Produkte wie beispielsweise Fruchtsäfte abgefüllt werden sollen. Weiterhin sind aus dem Stand der Technik diverse Vorrichtungen und Verfahren zum Sterilisieren der Behältnisse bekannt. So ist es beispielsweise bekannt, Behältnisse durch den Einsatz von Wasserstoffperoxid bzw. Peressigsäure zu sterilisieren. In jüngerer Zeit ist man jedoch bestrebt, auf den Einsatz von Chemikalien möglichst weitgehend zu verzichten. Daher sind aus dem Stand der Technik auch diverse Vorrichtungen und Verfahren bekannt, welche eine Sterilisation der Behältnisse bzw. der Behältnisoberflächen (innen wie außen) durch eine Bestrahlung durchführen. Auch ist es bekannt, diese Oberflächen insbesondere mit Elektronen zu bestrahlen, um auf diese Weise die Sterilisationseffekte zu erreichen.

Derartige Sterilisationsanlagen, welche zur Sterilisation Elektronenstrahlen einsetzen, weisen üblicherweise eine Elektronenerzeugungseinrichtung auf, sowie auch eine Beschleunigungseinrichtung, welche die Ladungsträger (insbesondere innerhalb eines Vakuums) in Richtung eines Austrittsfensters beschleunigt. Systembedingt ist es dabei oft erforderlich, dieses Austrittsfenster zu kühlen. Dabei wird üblicherweise Luft angesaugt, sterilisiert und anschließend den Austrittsfenstern zugeführt. Dabei muss die Kühlluft im Stand der Technik über einen Drehverteiler auf den drehenden Teil der Anlage gebracht werden, da sich diese Strahleinrichtungen üblicherweise auf einem beweglichen Träger wie einem Karussell befinden. Hierfür muss in dem Drehverteiler eine Medienspur für die Luft bereit gestellt sein. Da weiterhin eine sterile Ausführung derartiger Drehverteiler sehr kostenintensiv ist, befindet sich üblicherweise auch die Sterilluftfiltration auf einem entsprechenden Karussell. Nach der Filtration wird die Luft auf die einzelnen Stationen verteilt.

Die Strömung der Sterilluft muss dabei überwacht werden, da ein Ausfall einer Kühlung eine Überhitzung und damit eine Zerstörung des Austrittsfensters bzw. dessen Folie zur Folge hat. Weiterhin muss diese Kühlluft den Sterilisationseinrichtungen so zugeführt werden, dass auch der Strahlenschutz des Systems gegen die Umgebung gewährleistet ist.

Der vorliegenden Erfindung liegt daher die Aufgabe zugrunde, derartige Kühlungen des Austrittsfensters bzw. der Austrittsfenster zu vereinfachen. Dies wird erfindungsgemäß durch die Gegenstände der unabhängigen Ansprüche erreicht. Vorteilhafte Ausführungsformen und Weiterbildungen sind Gegenstand der Unteransprüche.

Das Verfahren gemäß der Erfindung ist durch Anspruch 1 definiert.

Erfindungsgemäß wird dieser Gasstrom zum Kühlen des Austrittsfensters wenigstens teilweise und bevorzugt vollständig dem Reinraum entnommen.

Während im Stand der Technik, wie oben beschrieben, die Kühlluft von außen, d. h. aus der Umgebung des Reinraums zugeführt wird, wird im Rahmen der Erfindung vorgeschlagen, diese Kühlluft direkt aus dem Reinraum bzw. Sterilraum (in dem sich bereits Sterilluft befindet) zu entnehmen. Dies bietet den Vorteil, dass bei entsprechender Abdichtung diese Sterilluft unbedenklich auch wieder dem Reinraum zugeführt werden kann, da sie letztlich aus diesem stammt. Daher ist es möglich, insoweit auf einen Drehverteiler, welcher Sterilluft führt, zu verzichten. Damit findet bevorzugt eine Art Umwälzung von Luft aus dem Reinraum und wieder als Kühlluft in den Reinraum statt. Bevorzugt wird die zum Kühlen des Austrittsfensters verwendete Luft wieder dem Reinraum zugeführt.

Bevorzugt handelt es sich bei den Behältnissen um Kunststoffbehältnisse wie beispielsweise Kunststoffflaschen oder Kunststoffvorformlinge. Besonders bevorzugt handelt es sich bei den Behältnissen um Kunststoffvorformlinge.

Mit anderen Worten wird die Sterilluft innerhalb des Reinraums bzw. Isolators der Sterilisationsvorrichtung genutzt, um die Austrittsfenster bzw. die Folien der Sterilisationseinrichtungen zu kühlen.

Bevorzugt wird der Gasstrom zum Kühlen des/der Austrittsfenster zunächst aus dem Reinraum heraus und anschließend wieder in den Reinraum hineingeführt. Auf diese Weise kann, insbesondere bei Vorhandensein mehrerer Sterilisationseinrichtungen eine günstige Verteilung auf die einzelnen Sterilisationseinrichtungen erfolgen, wobei derartige Verteileinheiten außerhalb des Reinraums angeordnet sein können.

Es wäre jedoch auch denkbar, den zur Kühlung der Austrittsfenster nötigen Gasstrom vollständig innerhalb des Reinraums zu belassen.

Bei dem Verfahren wird der Gasstrom wenigstens abschnittsweise mittels einer sich mit den Behältnissen bewegenden Verbindungsleitung geführt. So kann beispielsweise eine Zentralleitung in Form einer Ringleitung vorgesehen sein, welche mit der besagten Sterilluft aus dem Reinraum versorgt wird und welche ihrerseits wieder den einzelnen Austrittsfenstern die Kühlluft zuführt.

Bei einem weiteren bevorzugten Verfahren wird der Reinraum gegenüber der (unsterilen) Umgebung unter einen Überdruck gesetzt. Dieser Überdruck wird jedoch bevorzugt nicht durch den hier erwähnten Gasstrom erzeugt, da dieser Gasstrom bei einer Ausgestaltung dem Reinraum entzogen aber auch wieder zugeführt wird.

Vorteilhaft wird dabei dem Reinraum das zur Kühlung benötigte gasförmige Medium unter einen Überdruck von wenigstens 0,05 bar, bevorzugt von wenigstens 0,1 bar und besonders bevorzugt von wenigstens 0,15 bar zugeführt. Ein derartiger Überdruck kann beispielsweise ausreichen, um einen Soll-Volumen-Strom, der zur Kühlung der Sterilisationseinheiten verwendet wird, im Bereich von 20 l/min zu erzeugen.

Allgemein kann es sich bei dem Gasstrom um einen Luftstrom etwa einen Sterilluftstrom handeln. In diesem Falle befindet sich in dem Reinraum eine Atmosphäre aus Sterilluft. Es wäre jedoch auch möglich, eine Atmosphäre aus anderen Gasen in dem Reinraum vorzusehen wie etwa eine Stickstoff- Atmosphäre.

Bei einem weiteren bevorzugten Verfahren wird die dem Reinraum zur Kühlung der Austrittsfenster entnommene Luft bzw. der zur Kühlung der Austrittsfenster zu verwendende Gasstrom verdichtet. Zu diesem Zweck kann sich ein Verdichter beispielsweise auf einem sich mitbewegenden bzw. drehenden Maschinenteil und insbesondere außerhalb des Reinraums befinden.

Weiterhin ist es auch möglich, über eine Funktionsprüfung dieses Verdichters zu schließen, ob genügend Kühlluft vorhanden ist. Bevorzugt wird damit ein Volumenstrom, der den Austrittsfenstern zugeführt wird, gemessen. Dabei ist es auch möglich, dass, falls festgestellt wird, dass der Volumenstrom nicht ausreichend sein sollte, die entsprechenden Sterilisationseinrichtungen abgeschaltet werden, um so die teuren Austrittsfenster zu schützen. Besonders bevorzugt erfolgt die Funktionsprüfung über einen (einfachen) Druckaufnehmer stromabwärts des Verdichters.

Dadurch, dass die Kühlluft nicht mehr von außen in das System geführt werden muss, entfallen auch konstruktiv aufwendige Strahlenschutzmaßnahmen, die beispielsweise bei Leitungsdurchführungen notwendig wären sowie lange Leitungswege, die die Sterilisation des Systems erschweren. Auch kann auf den oben erwähnten Mediendrehverteiler zum Zuführen der Kühlluft verzichtet werden. Daneben kann auch über eine Funktionsprüfung des oben erwähnten Verdichters eine Aussage getroffen werden, ob ausreichend Kühlluft vorhanden ist. Auch eine aufwendige Überwachung wie beispielsweise durch Massendurchflussmesser oder Flowswitches kann verzichtet werden. Damit dient vorteilhaft der Verdichter auch zur Überwachung eines Volumenstroms, welcher den Austrittsfenstern zugeführt wird.

Bevorzugt ist es auch möglich, während einer Sterilisationsphase (insbesondere außerhalb eines Arbeitsbetriebs) den Reinraum unter eine Sterilisationsmittelatmosphäre (beispielsweise eine H₂O₂-Atmosphäre) zu setzten, wobei vorteilhaft diese Atmosphäre auch über den oben erwähnten Verdichter geführt werden kann und damit auch zur Sterilisation des erwähnten Kühlluftweges verwendet werden kann.

Bevorzugt wird die dem Reinraum entnommene Luft (bzw. allgemein das entnommene gasförmige Medium) temperiert und insbesondere gekühlt, bevor sie den einzelnen Austrittsfenstern zugeführt wird. Dabei können zum Kühlen dieser Luft Wärmetauscher, wie an sich aus dem Stand der Technik bekannt, verwendet werden.

Weiterhin wäre es möglich, dass dieses gasförmige Medium von mehreren Kühlaggregaten gekühlt wird. Dabei können mehrere Kühlaggregate hintereinander angeordnet sein und/oder auch redundant ausgeführt sein. Auch können derartige Kühlaggregate nach unterschiedlichen technischen Prinzipien arbeiten, so z.B. wenigstens ein Wärmetauscher und wenigstens ein Peltier-Element zur Kühlung vorgesehen sein. Bei einer weiteren vorteilhaften Ausführungsform bewegt sich wenigstens eine Einrichtung zum Temperieren und insbesondere Kühlen des gasförmigen Mediums mit den Behältnissen mit bzw. ist besonders bevorzugt an einem beweglichen Teil der Reinraumwandung angeordnet.

Die vorliegende Erfindung ist weiterhin auf eine Vorrichtung zum Sterilisieren von Behältnissen nach Anspruch 6 gerichtet.

Erfindungsgemäß weist die Vorrichtung eine Zuführeinrichtung auf, welche dem Austrittsfenster zu dessen Kühlung ein gasförmiges Medium aus dem Reinraum zuführt. Vorteilhaft wird zur Kühlung des oder der Austrittsfenster ausschließlich ein gasförmiges Medium aus dem Reinraum verwendet.

Es wird daher auch vorrichtungsseitig vorgeschlagen, dass die Luft, welche zur Kühlung der Austrittsfenster dient, aus dem Reinraum entnommen wird. Ein weiterer Vorteil der hier vorgeschlagenen Vorgehensweise bzw. einer entsprechenden Ausführungsform der Vorrichtung besteht darin, dass eine Luftbilanz der Anlage verbessert wird, da ein zugeführter Volumen-strom auch wieder abgeführt wird. Falls gewünscht, kann im Rahmen der vorliegenden Erfindung auch eine Strömung der Sterilluft innerhalb des Reinraums erzeugt werden, etwa, wenn die Sterilluft nach unten hin aus dem Reinraum entnommen wird und wieder von oben her in den Reinraum zur Kühlung der Austrittsfenster eingeführt wird.

Vorteilhaft weist diese Zuführeinrichtung wenigstens eine Verbindungsleitung auf, wobei ein Ende dieser Verbindungsleitung in den Reinraum mündet.

Vorteilhaft weist die Transporteinrichtung einen drehbaren Träger auf. An diesem drehbaren Träger kann eine Vielzahl der erwähnten Sterilisationseinrichtungen angeordnet sein. Vorteilhaft weisen die einzelnen Sterilisationseinrichtungen jeweils Beschleunigungseinrichtungen auf, welche die erzeugten Ladungsträger in Richtung des Austrittsfensters beschleunigen.

Bei einer weiteren vorteilhaften Ausführungsform weist der Reinraum eine Druckbeaufschlagungseinrichtung auf, welche den Reinraum unter einen Überdruck versetzt. Dieser Überdruck kann auch dazu dienen, um ausreichend Kühlluft zur Kühlung der Austrittsfenster zur Verfügung zu stellen. Bei einer weiteren vorteilhaften Ausführungsform weist die Vorrichtung eine Verdichtereinrichtung, welche aus dem Reinraum stammende Luft bzw. den Gasstrom verdichtet. Vorteilhaft ist diese Verdichtereinrichtung außerhalb des Reinraums angeordnet.

Weiterhin ist vorteilhaft auch eine Erfassungseinheit vorgesehen, die - insbesondere aus einer Funktion der Verdichtereinrichtung - auf eine Durchflussmenge der Kühlluft schließt. Es wäre jedoch auch möglich, dass zusätzlich eine Sensoreinrichtung oder eine Erfassungseinheit vorgesehen ist, welche eine Durchflussmenge des dem Reinraum entnommenen Gases bestimmt.

Bei einer weiteren vorteilhaften Ausführungsform weist die Sterilisationseinrichtung einen stangenartigen Körper auf, an dessen Ende das oben erwähnte Austrittsfenster angeordnet ist. Dabei kann dieser stangenartige Formkörper derart ausgebildet sein, dass er in das Innere der zu sterilisierenden Behältnisse einführbar ist um so deren Innenwandungen zu sterilisieren. Vorteilhaft weist dabei die Sterilisationseinrichtung bzw. dieser stangenartige Körper der in das Innere des Behältnisses einführbar ist, Kühlluftkanäle auf, um die Kühlluft an das untere Ende dieses stangenartigen Körpers zu fördern.

So kann beispielsweise ein Gehäuse dieses stangenartigen Körpers einen Ringspalt aufweisen, der zur Zuführung der Kühlluft an das Austrittsfenster dient. Dieser Ringspalt kann wiederum wenigstens zwei bevorzugt wenigstens drei Austrittsöffnungen aufweisen, über welche Kühlluft auf das Austrittsfenster geleitet wird und diese kühlt. Vorteilhaft wird dabei die Kühlluft in einem Bereich der Sterilisationseinrichtung umgelenkt, sodass sie von außen bzw. schräg auf das Austrittsfenster auftrifft.

Vorteilhaft ist der gesamte Luftströmungsweg insbesondere auch bis zu dem Austritt auf das Austrittsfenster sterilisierbar.

Vorteilhaft weist die oben erwähnte Zuführeinrichtung eine Verbindungsleitung auf, welche wenigstens teilweise außerhalb des Reinraums verläuft. Vorteilhaft ist dabei diese Verbindungsleitung beweglich angeordnet und bewegt sich vorteilhaft mit einem Träger bzw. der Transporteinrichtung, welche die Behältnisse mit befördert.

Bei einer weiteren vorteilhaften Ausführungsform ist der Reinraum von wenigstens zwei Wandungen abgegrenzt, die bevorzugt bezüglich einander beweglich sind. So kann beispielsweise eine Dichtungseinrichtung etwa in Form einer hydraulischen Dichtung wie eines Wasserschlosses vorgesehen sein, welche die beiden bezüglich einander beweglichen Wandungen gegeneinander abdichtet. Alternativ und besonders bevorzugt ist die Dichtungseinrichtung als trockener "Grauraum" in Form beispielsweise eines Absaugkanals ausgebildet. Dadurch wird vorteilhaft die Bildung von salpetriger Säure durch die Elektronenbestrahlung von feuchter Luft unterbunden.

Vorteilhaft ist dabei die oben erwähnte Verbindungsleitung an einer beweglichen Wandung, welche den Reinraum abgrenzt, angeordnet.

Bei einer weiteren vorteilhaften Ausführungsform weist die Vorrichtung eine Temperiereinrichtung und insbesondere eine Kühleinrichtung auf, welche die dem Reinraum entnommene und dem Austrittsfenster zuzuführende Luft bzw. den Gasstrom temperiert. Insbesondere kühlt dabei diese Temperiereinrichtung die Kühlluft. Bei dieser Temperiereinrichtung kann es sich beispielsweise um einen Wärmetauscher handeln. Vorteilhaft ist dabei in einer noch zentralen Leitung dieser Wärmetauscher angeordnet und erst stromabwärts dieses Wärmetauschers wird die Luft auf die einzelnen Sterilisationseinrichtungen aufgeteilt.

Vorteilhaft ist auch diese Temperiereinrichtung außerhalb des Reinraums angeordnet.

Bei einer weiteren vorteilhaften Ausführungsform ist wenigstens eine Ventileinrichtung und/oder eine Steuerungseinrichtung vorgesehen, welche die Zufuhr des gasförmigen Mediums in die oben erwähnte Verbindungsleitung steuern kann. So kann, falls gewünscht, die Zufuhr in die Verbindungsleitung (beispielsweise zu Reinigungszwecken) abgeschaltet werden.

Bei einer weiteren vorteilhaften Ausführungsform ist dem Reinraum auch ein gasförmiges Sterilisationsmedium zuführbar und dieses ist bevorzugt auch der besagten Verbindungsleitung zuführbar.

Bei einer weiteren vorteilhaften Ausführungsform weist die Vorrichtung eine Reinigungseinrichtung zum Reinigen von innerhalb des Reinraums angeordneten Elementen der Vorrichtung auf. So können beispielsweise an beweglichen oder stehenden bzw. drehenden oder stehenden Teilen der Anordnung jeweils Beaufschlagungseinrichtungen vorgesehen sein, welche zum Beaufschlagen der besagten Elemente mit einem Reinigungsmittel dienen.

Weitere Vorteile und Ausführungsformen ergeben sich aus den beigefügten Zeichnungen:

Darin zeigen:
- Fig. 1: Eine perspektivische Darstellung einer Sterilisationsvorrichtung;
- Fig. 2: Eine grob schematische Darstellung einer einzelnen Sterilisationseinrichtung;
- Fig. 3: Eine Darstellung eines unteren Abschnittes der in Figur 2 gezeigten Sterilisationseinrichtung;
- Fig. 4a: Eine schematische Darstellung einer Vorrichtung nach dem Stand der Technik;
- Fig. 4b: Eine vereinfachte schematische Darstellung einer erfindungsgemäßen Anordnung; und
- Fig. 5: Eine detailliertere Zeichnung einer erfindungsgemäßen Anordnung.

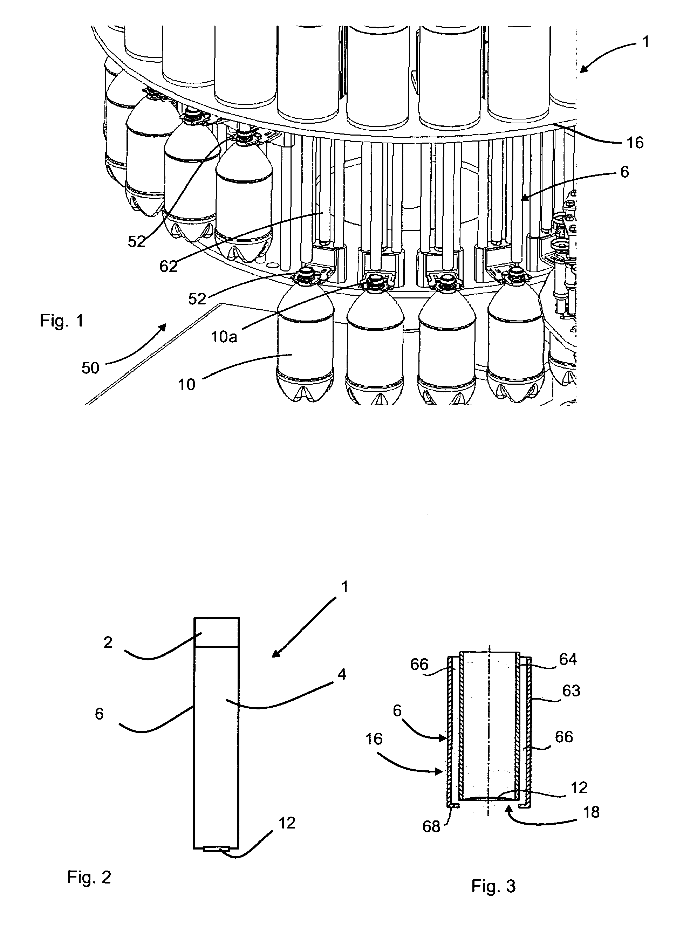

Figur 1 zeigt eine perspektivische vereinfachte Darstellung einer Vorrichtung 50 zum Sterilisieren von Behältnissen. Dabei ist ein drehbarer Träger 16 vorgesehen, an dem eine Vielzahl von Sterilisationseinrichtungen 1 angeordnet ist. Dieser Träger 16 stellt damit einen Teil einer Transporteinrichtung zum Transportieren der Behältnisse 10 dar. Vorteilhaft ist ein Antrieb, welcher den Träger antreibt, außerhalb des unten genauer beschriebenen Reinraums 20 angeordnet.

Dabei weisen diese Sterilisationseinrichtungen 1 jeweils stangenartige Körper 6 auf, in denen die Ladungsträger zu einem (nicht gezeigten) am unteren Ende befindlichen Austrittsfenster geleitet werden. Diese stangenartigen Körper 6, die auch jeweils als Gehäuse ausgebildet sind, können dabei über Mündungen 10a in Behältnisse 10 eingeführt werden. In Figur 1 sind als Behältnisse Kunststofflaschen dargestellt, die hierbeschriebene Vorrichtung sowie das beschriebene Verfahren eignen sich jedoch auch insbesondere zur (Innen)sterilisation von Kunststoffvorformlingen.

Das Bezugszeichen 62 bezieht sich auf eine Bewegungseinrichtung, an deren unterem Ende eine Halteeinrichtung 52 zum Halten der Behältnisse 10 angeordnet ist. Hier greift diese Halteeinrichtung den Mündungsbereich 10a der Behältnisse, insbesondere unterhalb des Tragrings. Diese Bewegungseinrichtung 62 kann dabei, wie in Figur 1 gezeigt, die Behältnisse nach oben anheben und damit den Strahlfinger 6 in das Innere der Behältnisse 10 einführen.

Figur 2 zeigt eine vereinfachte schematische Darstellung einer Sterilisationseinrichtung 1. Diese Sterilisationseinrichtung 1 weist dabei wiederum eine Ladungsträgererzeugungseinrichtung 2 auf, sowie eine (nur schematisch dargestellte) Beschleunigungseinrichtung 4, welche die Ladungsträger innerhalb des Strahlfingers bzw. des Gehäuses 6 in Richtung eines Austrittsfensters 12 beschleunigt. Dieses Austrittsfenster 12 ist dabei bevorzugt ein Titanfenster, welches besonders bevorzugt eine Dicke aufweist, welche zwischen 5µm und 20µm liegt.

Figur 3 zeigt einen unteren Abschnitt des Strahlfingers bzw. des Gehäuses 6 und insbesondere die Vorrichtungen zu dessen Kühlung. Dabei ist hier wenigstens ein Kühlluftkanal 66 vorgesehen, um Kühlluft in Richtung des Austrittsfensters 12 zu führen. Weiterhin ist hier eine Umlenkeinrichtung 68 (nur grob schematisch dargestellt) vorgesehen, welche die Kühlluft auf das Austrittsfenster 12 leitet. Der Strahlfinger weist hier ein inneres Gehäuse 64 sowie ein äußeres Gehäuse 63 auf, wobei zwischen diesen Gehäusen 63, 64 die Strömungswege für die Kühlluft ausgebildet sind.

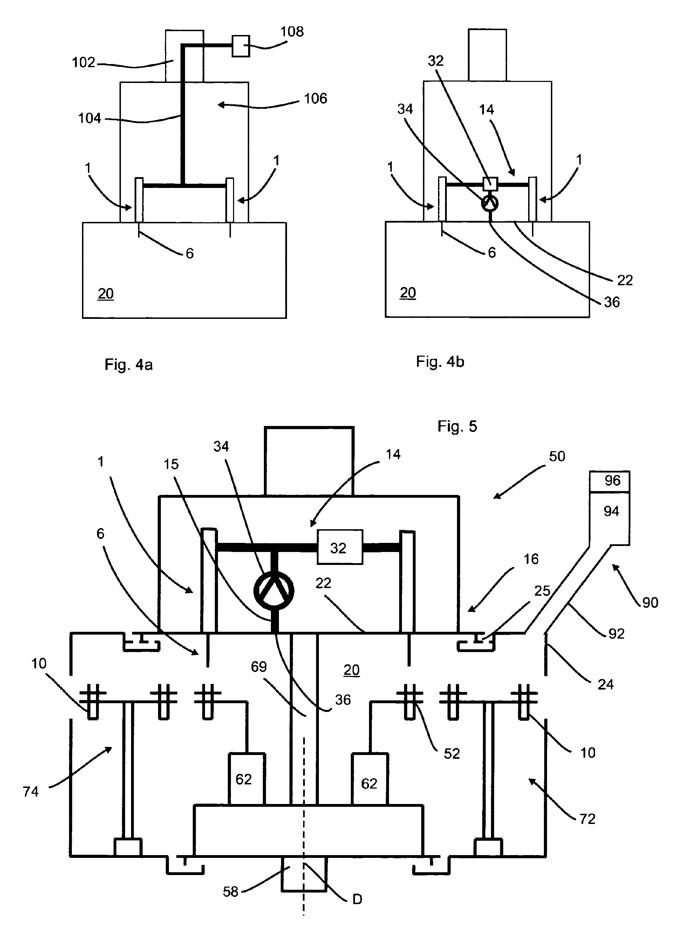

Figur 4a zeigt eine Vorrichtung nach dem Stand der Technik. Hier ist grob schematisch wiederum der Reinraum 20 dargestellt, innerhalb dessen die einzelnen Strahlfinger 6 angeordnet sind und innerhalb dessen auch die (nicht gezeigten) Behältnisse transportiert werden. Weitere Teile der Sterilisationseinrichtung wie beispielsweise die Beschleunigungseinrichtung und die Ladungsträgererzeugungseinrichtung sind außerhalb des Reinraums 20 angeordnet. Die Kühlluft muss hier mit einem Drehverteiler 102 auf den drehenden Teil 106 der Anlage über eine Zuleitung 104 gebracht werden, da sich die einzelnen Sterilisationseinrichtungen 1 auf einem Karussell befinden. Nach einer Filtration wird die Sterilluft auf die einzelnen Sterilisationseinrichtungen innerhalb des Karussells verteilt. Das Bezugszeichen 106 bezieht sich dabei genauer auf einen drehenden Teil in der Anlage, der jedoch außerhalb des Reinraums 20 angeordnet ist. Das Bezugszeichen 108 kennzeichnet eine Sterilluftquelle.

Figur 4b zeigt eine schematische Darstellung einer erfindungsgemäßen Anordnung. Man erkennt hier, dass die Kühlluft nicht von außen über den Drehverteiler herbeigeführt wird, sondern aus dem Reinraum 20 entnommen wird und anschließend über einen Verdichter 34 und eine Kühleinrichtung 32 geführt wird um so wiederum auf die einzelnen Sterilisationseinrichtungen 1 verteilt zu werden. Das Bezugszeichen 14 kennzeichnet in seiner Gesamtheit die Zuführeinrichtung welche zum Zuführen der Kühlluft an die einzelnen Austrittsfenster dient. Das Bezugszeichen 36 kennzeichnet eine Öffnung des Reinraums, über welche die Sterilluft aus dem Reinraum 20 abgeführt werden kann. Es wäre dabei auch möglich, mehrere derartiger Öffnungen vorzusehen. Daneben könnte in der von der Öffnung weiterführenden Leitung auch ein Rückschlagventil oder ein ähnliches Ventil vorgesehen sein, welches ein Rückströmen von Luft in den Reinraum 20 verhindert.

Figur 5 zeigt eine detailliertere Darstellung einer erfindungsgemäßen Vorrichtung 50. Auch hier ist der Träger bzw. die Transporteinrichtung 16 dargestellt (und die hier auch die Wandung 22 ausbildet), welche hier einen Antrieb 58 aufweist, der eine Drehung bezüglich einer Drehachse D erzeugt. An dieser Transporteinrichtung 16 ist eine Vielzahl von Hubeinrichtungen 62 vorgesehen, welche die einzelnen Kunststoffvorformlinge 10 hier in Richtung der Drehachse D anheben oder absenken können. Dabei wäre es denkbar, dass es sich bei diesen Hubeinrichtungen um elektromotorische Antriebe handelt. Denkbar wären jedoch auch hydraulische, pneumatische oder magnetische Antriebe oder auch Hubkurven. Auch wäre es möglich, dass diese Hubeinrichtungen zumindest teilweise außerhalb des Reinraums 20 angeordnet sind.

Weiterhin sind oberhalb dieser Hubeinrichtungen 62 wieder die einzelnen Sterilisationseinrichtungen 1 angeordnet wobei eine Wandung 22, an der diese befestigt sind, auch eine Begrenzung des Reinraums 20 ausbildet. Diese Wandung 22 dreht sich hier gegenüber einer stationären Wandung 24. Das Bezugszeichen 25 bezieht sich auf eine Abdichteinrichtung, welche die rotierende Wandung 22 gegenüber der stehenden Wandung 24 abdichtet. Das Bezugszeichen 69 kennzeichnet eine Zentralwelle, die zum Übertragen der Drehbewegung auch auf die Trägerwandung 22 mit den einzelnen Sterilisationseinrichtungen 1 dient. Die stangenartigen Körper 6 ragen dabei permanent in das Innere des Reinraums 20 und sind bevorzugt fest an dem Träger 16 angeordnet.

Das Bezugszeichen 72 kennzeichnet eine Zuführeinrichtung wie einen Zuführstern, der die Kunststoffvorformlinge der Transporteinrichtung 16 zuführt und das Bezugszeichen 74 kennzeichnet eine entsprechende Abführeinrichtung.

Über eine Leitung 15 wird die Kühlluft aus dem Reinraum 20 entnommen, über die Verdichtungseinrichtung 34 verdichtet und anschließend wie oben erwähnt den einzelnen Sterilisationseinrichtungen 1 zugeführt. Das Bezugszeichen 32 kennzeichnet wie oben erwähnt eine Kühleinrichtung zum Kühlen der Kühlluft.

Das Bezugszeichen 90 bezieht sich in seiner Gesamtheit auf eine Druckbeaufschlagungseinrichtung, welche dem Sterilraum Sterilluft zuführen kann. Diese Druckbeaufschlagungseinrichtung 90 kann dabei eine Zuführleitung 92 aufweisen, welche bevorzugt in einer stationäre Wandung 24 des Reinraums 20 mündet und welche dem Reinraum 20 auf diese Weise Sterilluft zuführen kann und welche bevorzugt den Reinraum auch unter einen Überdruck setzen kann. Das Bezugszeichen 94 kennzeichnet eine Filtereinrichtung zum Herstellen der Sterilluft, wie etwa einen HEPA- Filter. Bevorzugt ist auch diese Filtereinrichtung stationär angeordnet. Das Bezugszeichen 96 kennzeichnet eine Luftquelle wie etwa einen Kompressor.

### Bezugszeichenliste

- 1: Sterilisationseinrichtung
- 2: Ladungsträgererzeugungseinrichtung,
- 4: Beschleunigungseinrichtung
- 6: stangenartige Körper, Strahlfinger bzw. Gehäuse
- 10: Behältnisse; Kunststoffvorformlinge
- 10a: Mündungen
- 12: Austrittsfenster
- 14: Zuführeinrichtung
- 15: Leitung
- 16: drehbarer Träger, Transporteinrichtung
- 20: Reinraum
- 22: bewegliche Wandung
- 24: stationäre Wandung
- 25: Dichtungseinrichtung
- 32: Kühleinreichung
- 34: Verdichtungseinrichtung
- 36: Öffnung
- 50: Sterilisationsvorrichtung
- 52: Halteeinrichtung
- 58: Antrieb
- 62: Bewegungseinrichtung; Hubeinrichtung
- 63: äußeres Gehäuse
- 64: inneres Gehäuse
- 66: Kühlluftkanal
- 68: Umlenkeinrichtung
- 69: Zentralwelle
- 72: Zuführeinrichtung
- 74: Abführeinrichtung
- 90: Druckbeaufschlagungseinrichtung
- 92: Zuführleitung
- 94: Filtereinrichtung
- 96: Luftquelle
- 102: Drehverteiler (Stand der Technik)
- 104: Zuleitung (Stand der Technik)
- 106: drehender Teil in der Anlage (Stand der Technik)
- 108: Sterilluftquelle (Stand der Technik)

- D: Drehachse

## Patentansprüche

1. Verfahren zum Sterilisieren von Behältnissen (10), wobei die Behältnisse (10) entlang eines vorgegebenen Transportpfads durch einen Reinraum (20) transportiert werden, wobei dieser Reinraum (20) mittels wenigstens einer Wandung (22, 24) gegenüber einer Umgebung abgegrenzt wird und die Behältnisse (10) wenigstens abschnittsweise durch Beaufschlagung mit Elektronen sterilisiert werden, wobei die Elektronen erzeugt werden und über ein Austrittsfenster (12) einer Sterilisationseinrichtung (1) auf die Behältnisse gelangen und wobei dieses Austrittsfenster (12) mittels eines Gasstroms gekühlt wird und der Gasstrom wenigstens abschnittsweise mittels einer sich mit den Behältnissen (10) bewegenden Verbindungsleitung (14) geführt wird,
**dadurch gekennzeichnet, dass**
dieser Gasstrom zum Kühlen des Austrittsfensters (12) dem Reinraum (20) entnommen wird.

2. Verfahren nach Anspruch 1,
**dadurch gekennzeichnet, dass**
der Gasstrom zum Kühlen der Austrittsfenster (12) zunächst aus dem Reinraum (20) heraus und anschließend wieder in den Reinraum (20) hinein geführt wird.

3. Verfahren nach wenigstens einem der vorangegangenen Ansprüche,
**dadurch gekennzeichnet, dass**
der Reinraum (20) gegenüber der Umgebung unter einen Überdruck gesetzt wird.

4. Verfahren nach wenigstens einem der vorangegangenen Ansprüche,
**dadurch gekennzeichnet, dass**
dem Reinraum (20) das zur Kühlung der Austrittsfenster (12) benötigte gasförmige Medium unter einem Überdruck von wenigstens 0,05bar, bevorzugt von wenigstens 0,1 bar, bevorzugt von wenigstens 0,15bar zugeführt wird.

5. Verfahren nach wenigstens einem der vorangegangenen Ansprüche,
**dadurch gekennzeichnet, dass**
der dem Reinraum (20) zur Kühlung der Austrittsfenster (12) entnommene Gasstrom verdichtet wird.

6. Vorrichtung (50) zum Sterilisieren von Behältnissen (10) mit einer Transporteinrichtung (16) welche die Behältnisse (10) entlang eines vorgegebenen Transportpfades transportiert, mit einem Reinraum (20), der die transportierten Kunststoffbehältnisse (16) während ihres Transports umgibt, wobei dieser Reinraum (20) mittels wenigstens einer Wandung (22, 24) gegenüber einer Umgebung abgetrennt ist, mit einer Sterilisationseinrichtung (1) welche eine Elektronenerzeugungseinheit (2) aufweist, welche Elektronen erzeugt sowie ein Austrittsfenster (12) über welches die Elektronen aus einem Gehäuse (6) austreten können und wobei die Sterilisationseinrichtung (1) weiterhin eine Kühlungseinrichtung (16) zum Kühlen dieses Austrittsfensters (12) aufweist und diese Kühlungseinrichtung (16) eine Gasbeaufschlagungseinrichtung (18) aufweist, welche das Austrittsfenster (12) mit einem gasförmigen Medium beaufschlagt sowie eine sich mit den Behältnissen bewegende Verbindungsleitung (14) zum Führen des Gasstroms,
**dadurch gekennzeichnet, dass**
die Vorrichtung eine Zuführeinrichtung (14) aufweist, welche dem Austrittsfenster (12) zu dessen Kühlung Gas aus dem Reinraum (20) zuführt.

7. Vorrichtung nach Anspruch 6,
**dadurch gekennzeichnet, dass**
die Zuführeinrichtung (14) eine Verbindungsleitung (14) aufweist, welche wenigstens teilweise außerhalb des Reinraums (20) verläuft.

8. Vorrichtung nach Anspruch 7 oder 8,
**dadurch gekennzeichnet, dass**
der Reinraum (20) von wenigstens zwei Wandungen abgegrenzt ist, welche bezüglich einander beweglich sind.

9. Vorrichtung nach Anspruch 8,
**dadurch gekennzeichnet, dass**
die Verbindungsleitung (14) an einer beweglichen Wandung (22), welche den Reinraum (20) abgrenzt, angeordnet ist.

10. Vorrichtung (1) nach wenigstens einem der vorangegangenen Ansprüche 6 - 9,
**dadurch gekennzeichnet, dass**
die Vorrichtung (1) eine Temperiereinrichtung (32) aufweist, welche den dem Reinraum (20) entnommenen und dem Austrittsfenster (12) zuzuführenden Gasstrom temperiert.

## Claims

1. A method for sterilizing containers (10), wherein the containers (10) are conveyed along a pre-set conveying path through a clean room (20), wherein this clean room (20) is bounded off from an environment by means of at least one wall (22, 24) and the containers (10) are sterilized at least in sections by being acted upon with electrons, wherein the electrons are produced and arrive at the containers by way of an outlet window (12) of a sterilization device (1), and wherein this outlet window (12) is cooled by means of a gas flow and the gas flow is conveyed at least in sections by means of a connecting line (14) moving with the containers (10), **characterized in that** this gas flow for cooling the outlet window (12) is removed from the clean room (20) .

2. A method according to claim 1, **characterized in that** the gas flow for cooling the outlet windows (12) is first conveyed out of the clean room (20) and then into the clean room (20) again.

3. A method according to at least one of the preceding claims, **characterized in that** the clean room (20) is set at an over-pressure with respect to the environment.

4. A method according to at least one of the preceding claims, **characterized in that** the gaseous medium required for cooling the outlet windows (12) is supplied to the clean room (20) at an over-pressure of at least 0.05 bar, preferably of at least 0.1 bar, preferably of at least 0.15 bar.

5. A method according to at least one of the preceding claims, **characterized in that** the gas flow removed from the clean room (20) for cooling the outlet windows (12) is condensed.

6. An apparatus (50) for the sterilization of containers (10) with a conveying device (16) which conveys the containers (10) along a pre-set conveying path, with a clean room (20) which surrounds the conveyed plastics material containers (16) during the conveying thereof, wherein this clean room (20) is bounded off from an environment by means of at least one wall (22, 24), with a sterilization device (1) which has an electron production unit (2) which produces electrons and an outlet window (12) by way of which the electrons can issue from a casing (6), and wherein the sterilization device (1) additionally has a cooling device (16) for cooling this outlet window (12) and this cooling device (16) has a gasstressing device (18) which acts upon the outlet window (12) with a gaseous medium, and a connecting line (14) moving with the containers for conveying the gas flow, **characterized in that** the apparatus has a supply device (14) which supplies gas from the clean room (20) to the outlet window (12) for the cooling thereof.

7. An apparatus according to claim 6, **characterized in that** the supply device (14) has a connecting line (14) which extends at least in part outside the clean room (20).

8. An apparatus according to claim 6 or 7, **characterized in that** the clean room (20) is bounded off by at least two walls which are movable with respect to each other.

9. An apparatus according to claim 8, **characterized in that** the connecting line (14) is arranged on a movable wall (22) which bounds off the clean room (20).

10. An apparatus (1) according to at least one of the preceding claims 6 to 9, **characterized in that** the apparatus (1) has a tempering device (32) which tempers the gas flow removed from the clean room (20) and to be supplied to the outlet window (12).

## Revendications

1. Procédé de stérilisation de récipients (10), lesdits récipients (10) étant transportés au travers d'un espace stérile (20) le long d'un chemin de transport défini, ledit espace stérile (20) étant isolé d'un environnement par au moins une paroi (22, 24) et les récipients (10) étant au moins en sections stérilisés par bombardement d'électrons, lesdits électrons étant générés et parvenant aux récipients par une fenêtre de sortie (12) d'un dispositif de stérilisation (1), ladite fenêtre de sortie (12) étant refroidie par un flux gazeux et ledit flux gazeux étant au moins en sections guidé au moyen d'une conduite de raccordement (14) se déplaçant avec les récipients (10),
**caractérisé en ce que**
ledit flux gazeux est prélevé de l'espace stérile (20) pour le refroidissement de la fenêtre de sortie (12).

2. Procédé selon la revendication 1,
**caractérisé en ce que**
le flux gazeux pour le refroidissement des fenêtres de sortie (12) est d'abord refoulé hors de l'espace stérile (20), puis réintroduit dans l'espace stérile (20).

3. Procédé selon au moins une des revendications précédentes,
**caractérisé en ce que**
l'espace stérile (20) est soumis à une surpression par rapport à l'environnement.

4. Procédé selon au moins une des revendications précédentes,
**caractérisé en ce que**
le fluide gazeux nécessité pour le refroidissement de la fenêtre de sortie (12) est conduit vers l'espace stérile (20) avec une surpression d'au moins 0,05 bar, préférentiellement d'au moins 0,1 bar, préférentiellement d'au moins 0,15 bar.

5. Procédé selon au moins une des revendications précédentes,
**caractérisé en ce que**
le flux gazeux prélevé de l'espace stérile (20) pour le refroidissement de la fenêtre de sortie (12) est condensé.

6. Installation (50) pour la stérilisation de récipients (10), avec un dispositif de transport (16) convoyant les récipients (10) le long d'un chemin de transport défini, avec un espace stérile (20) qui entoure pendant leur transport les récipients en matière plastique (16) convoyés, ledit espace stérile (20) étant isolé d'un environnement par au moins une paroi (22, 24), avec un dispositif de stérilisation (1) pourvu d'une unité de génération d'électrons (2), laquelle génère des électrons et une fenêtre de sortie (12) par laquelle les électrons peuvent s'échapper d'un boîtier (6), et le dispositif de stérilisation (1) comportant en outre un dispositif de refroidissement (16) pour le refroidissement de ladite fenêtre de sortie (12) et ledit dispositif de refroidissement (16) comportant un dispositif d'application de gaz (18) qui applique un fluide gazeux sur la fenêtre de sortie (12), et une conduite de raccordement (14) se déplaçant avec les récipients pour le guidage du flux gazeux,
**caractérisée en ce que**
ladite installation comporte un dispositif d'amenée (14) qui conduit du gaz de l'espace stérile (20) vers la fenêtre de sortie (12) pour le refroidissement de celle-ci.

7. Installation selon la revendication 6,
**caractérisée en ce que**
le dispositif d'amenée (14) comporte une conduite de raccordement (14) qui s'étend au moins partiellement en dehors de l'espace stérile (20).

8. Installation selon la revendication 6 ou 7,
**caractérisée en ce que**
l'espace stérile (20) est isolé par au moins deux parois mobiles l'une par rapport à l'autre.

9. Installation selon la revendication 8,
**caractérisée en ce que**
la conduite de raccordement (14) est disposée contre une paroi mobile (22) isolant l'espace stérile (20).

10. Installation (1) selon au moins une des revendications 6 à 9,
**caractérisée en ce que**
ladite installation (1) comporte un dispositif de thermostatisation (32) qui régule la température du flux gazeux prélevé de l'espace stérile (20) et à conduire vers la fenêtre de sortie (12).
